# EUROPÄISCHE PATENTSCHRIFT

(11) **EP 1 032 320 B1**
(45) Veröffentlichungstag und Bekanntmachung des Hinweises auf die Patenterteilung: **03.03.2004**
(21) Anmeldenummer: 97912009.4
(22) Anmeldetag: 21.11.1997
(51) Int. Cl.: A61B 17/58, A61B 17/70

(54) **VORRICHTUNG ZUR SIMULATION VON UNTER DER HAUT POSITIONIERTEN IMPLANTATEN**
DEVICE FOR SIMULATING IMPLANTS LOCATED UNDER THE SKIN
DISPOSITIF DE SIMULATION D'IMPLANTS POSITIONNES SOUS LA PEAU

(43) Veröffentlichungstag der Anmeldung: 06.09.2000
(73) Patentinhaber: SYNTHES AG CHUR, 7002 Chur (CH)
(72) Erfinder: TRAXEL, Doris, CH-4052 Basel (CH); WEBER, Urs, CH-4418 Reigoldswil (CH)
(74) Vertreter: Lusuardi, Werther Giovanni, Dr.
(86) Internationale Anmeldenummer: PCT/CH1997/000440
(87) Internationale Veröffentlichungsnummer: WO 1999/026548

(56) Entgegenhaltungen:
- GB-A- 2 267 757
- US-A- 5 658 286

## Beschreibung

Die Erfindung bezieht sich auf eine Vorrichtung zur Simulation der Lage und Orientierung von mehreren zu einem Fixationssystem gehörenden Implantaten gemäss dem Oberbegriff des Patentanspruchs 1 und auf ein Verfahren zum Anbiegen eines Längsträgers gemäss dem unabhängigen Patentanspruch 16.

In der Chirurgie wird zur Reposition und/oder Stabilisierung der Wirbelsäule oder anderer frakturierter Knochen ein Implantatsystem in der Weise implantiert, dass die Wirbelsäule oder die Knochen weitgehend freigelegt werden und es dadurch zu einer massiven Schädigung der Weichteile und hier insbesondere der Muskulatur kommt. Diese Schädigung führt einerseits zu einer Schwächung im muskulären Bereich und damit zu einer Schwächung im Haltesystem für die Wirbelsäule oder anderen Extremitäten und birgt andererseits die Gefahr der Narbenbildung, die insbesondere an der Wirbelsäule beim Patienten zu postoperativen Problemen führen kann, in sich.

Ein Verfahren zur Anpassung eines Längsträgers an die durch die Position der Knochenfixationselemente bestimmte Form wird in der US 5,658,286 SAVA offenbart. Der Längsträger wird in-situ angepasst und mittels eines aushärtbaren Füllmaterials in der definitiven Form versteift. Nachteilig an diesem Verfahren ist, dass diese Anpassung der Form unter der Haut und/oder der Muskulatur und somit ohne Sichtkontakt erfolgt.

Hier will die Erfindung Abhilfe schaffen. Der Erfindung liegt die Aufgabe zugrunde, eine Vorrichtung zu schaffen, bei welcher das Implantat durch bzw. unter der Haut und/oder Muskulatur implantiert werden kann und die posteriore Rückenmuskulatur bzw. Muskulatur weitgehend intakt bleibt. Zu diesem Zweck werden Pedikelschrauben oder andere Implantate durch die Haut hindurch gesetzt, was bevorzugt mit Hilfe eines Computer Assisted Surgery System (CAS), wie es beispielsweise in der Patentschrift US 5,383,454 BUCHHOLZ dargestellt ist, geschieht. Die Pedikelschrauben und/oder anderen Implantate sind mit Verlängerungsstiften versehen, welche es ermöglichen, die Pedikelschrauben und/oder anderen Implantate durch die Haut in der Tiefe der Wirbelsäule oder der Knochen zu implantieren. Die Enden dieser Verlängerungsstifte ragen aus der Haut heraus. Nach dem Positionieren des Implantates können die Verlängerungstifte wieder entfernt werden. Ein wesentliches Problem liegt nun darin, dass die Stellung der Pedikelschrauben und/oder anderen Implantate am/im Knochen nicht sichtbar ist und eine Anmodellierung des Längsträgers mit einem Probestab respektive einer Biegeschablone wie bisher nicht mehr möglich ist. Die Anpassung des Längsträgers erfolgt nun mit Hilfe der erfindungsgemässen Vorrichtung, wobei vorgängig gleiche Implantate und gleiche Verlängerungsstifte, wie sie am Fixationssystem in situ angebracht sind in die erfindungsgemässe Vorrichtung eingefügt und in dieselbe Lage und Orientierung zueinander gebracht werden, wie sie am Fixationssystem angebracht sind. Ist die Position und Orientierung des gesamten Implantates in der erfindungsgemässen Vorrichtung identisch wie in situ wird ein Probestab respektive eine Biegeschablone in die dazu vorgesehenen Öffnungen der Implantate in der erfindungsgemässen Vorrichtung gelegt und angepasst. Der Längsträger wird dann wie bei der konventionellen Methode mechanisch oder elektronisch angepasst. Der Chirurg hat weiterhin die Möglichkeit, den Stab entsprechend einer gewünschten Korrektur anzubiegen. Dadurch, dass die Querträger der erfindungsgemässen Vorrichtung eine gewisse Flexibilität aufweisen, lässt sich dann auch simulieren, wie die Anatomie verändert wird, wenn der Stab mit leichten Veränderungen gegenüber der durch die Implantate in situ gegebenen Form gebogen wird.

Die Erfindung löst die gestellte Aufgabe mit einer Vorrichtung zur Simulation der Lage und Orientierung von mehreren zu einem Fixationssystem gehörenden Implantaten, welche die Merkmale des Anspruchs 1 aufweist, sowie einem Verfahren zum Anbiegen eines Längsträgers gemäss dem unabhängigen Patentanspruch 16.

Eine vorteilhafte Ausführungsform der erfindungsgemässen Vorrichtung besteht darin, dass die erfindungsgemässe Vorrichtung eine Grundplatte mit parallel angebrachten Längsschienen, verschiebbare Querträger mit integrierten Kugelgelenken und eine Positioniereinrichtung umfasst. Die Querträger lassen sich in den zwei Dimensionen der Grundplattenebene bewegen und durch Klemmschrauben in einer beliebigen Position fixieren. In die erfindungsgemässe Vorrichtung werden dann die gleichen Implantate mit gleichen Verlängerungsstiften wie im Fixationssystem in situ eingeschraubt. Eine zur Simulationseinrichtung gehörende Positioniereinrichtung umfasst an den Verlängerungsstiften angebrachte Light Emitting Dioden (LED), mehrere diese emittierten elektromagnetischen Wellen detektierende Sensoren im Raum und eine Auswerteeinheit. Die Auswerteeinheit ermöglicht durch computerunterstützte Verarbeitung von Interferenzbildern, dass die Verlängerungsstifte und die Implantate in der erfindungsgemässen Vorrichtung so positioniert werden können, dass sie dieselbe Lage und Orientierung zueinander einnehmen wie die am Fixationssystem angebrachten. Ist dann die Position der Implantate und der Verlängerungsstifte in der erfindungsgemässen Vorrichtung identisch wie in situ wird ein Probestab respektive eine Biegeschablone in die dazu vorgesehenen Öffnungen an den Köpfen der Implantate in der erfindungsgemässen Vorrichtung gelegt und angepasst oder die Position der Implantate zueinander wird elektronisch vermessen und der Längsträger mechanisch angebogen.

Eine andere vorteilhafte Ausführungsform der erfindungsgemässen Vorrichtung besteht darin, dass die zur erfindungsgemässen Vorrichtung gehörende Positioniereinrichtung an den Verlängerungsstiften angebrachte elektromagnetischen Wellen reflektierende Mittel, mindestens ein elektromagnetische Wellen emittierendes Mittel im Raum, mehrere die reflektierten Wellen detektierende Sensoren im Raum und eine Auswerteeinheit umfasst. Die Verarbeitung der detektierten Signale mittels der Auswerteeinheit erfolgt analog zu der oben aufgeführten Ausführungsform der erfindungsgemässen Vorrichtung.

Eine andere vorteilhafte Ausführungsform der erfindungsgemässen Vorrichtung besteht darin, dass die zur erfindungsgemässen Vorrichtung gehörende Positioniereinrichtung an den Verlängerungsstiften angebrachte Hall-Sensoren, mindestens eine ein Magnetfeld aufbauende Spule im Raum und eine Auswerteeinheit umfasst. Die Verarbeitung der detektierten Signale mittels der Auswerteeinheit erfolgt analog zu der oben aufgeführten Ausführungsform der erfindungsgemässen Vorrichtung.

Eine andere vorteilhafte Ausführungsform der erfindungsgemässen Vorrichtung besteht darin, dass die zur erfindungsgemässen Vorrichtung gehörende Positioniereinrichtung an den Verlängerungsstiften angebrachte ein Magnetfeld aufbauende Spulen, Hall-Sensoren zur Detektierung des Magnetfeldes im Raum und eine Auswerteeinheit umfasst. Die Verarbeitung der detektierten Signale mittels der Auswerteeinheit erfolgt analog zu der oben aufgeführten Ausführungsform der erfindungsgemässen Vorrichtung.

Wieder eine andere vorteilhafte Ausführungsform der erfindungsgemässen Vorrichtung besteht darin, dass die Sensoren der Positioniereinrichtung Kameras sind. Mit Hilfe von diesen Kameras können die von den Sendern, welche an den Verlängerungsstiften angebracht sind, emittierten elektromagnetischen Wellen aufgenommen werden. Die zugehörige Auswerteeinheit ermöglicht, die durch die Kameras aufgenommenen Bilder videogrammetrisch zu verarbeiten, so dass die in der erfindungsgemässen Vorrichtung eingespannten Verlängerungsstifte und Implantate in dieselbe Lage und Orientierung zueinander gebracht werden können wie die zum Fixationssystem in situ gehörenden Implantate und Verlängerungsstifte. Ist dann die Position der Implantate und der Verlängerungsstifte in der erfindungsgemässen Vorrichtung identisch wie in situ wird ein Probestab respektive eine Biegeschablone in die dazu vorgesehenen Öffnungen an den Köpfen der Implantate in der erfindungsgemässen Vorrichtung gelegt und angepasst oder die Position der Implantate zueinander wird elektronisch vermessen und der Längsträger mechanisch angebogen.

Wiederum eine andere vorteilhafte Ausführungsform der erfindungsgemässen Vorrichtung besteht darin, dass die zur erfindungsgemässen Vorrichtung gehörende Positioniereinrichtung an den Verlängerungsstiften angebrachte Ultraschallwellen detektierende Sensoren, mindestens einen Ultraschallwellen emittierenden Sender im Raum und eine Auswerteeinheit umfasst. Die Auswerteeinheit ermöglicht durch Verarbeitung von Interferenzmustern, dass die Verlängerungsstifte und die Implantate in der erfindungsgemässen Vorrichtung so positioniert werden können, dass sie dieselbe Lage und Orientierung zueinander einnehmen wie die am Fixationssystem angebrachten. Ist dann die Position der Implantate und der Verlängerungsstifte in der erfindungsgemässen Vorrichtung identisch wie in situ wird ein Probestab respektive eine Biegeschablone in die dazu vorgesehenen Öffnungen an den Köpfen der Implantate in der erfindungsgemässen Vorrichtung gelegt und angepasst oder die Position der Implantate zueinander wird elektronisch vermessen und der Längsträger mechanisch angebogen.

Wieder eine andere vorteilhafte Ausführungsform der erfindungsgemässen Vorrichtung besteht darin, dass die zur erfindungsgemässen Vorrichtung gehörende Positioniereinrichtung an den Verlängerungsstiften angebrachte Ultraschallwellen emittierende Sender, mindestens zwei Ultraschallwellen detektierende Sensoren im Raum und eine Auswerteeinheit umfasst. Die Verarbeitung der detektierten Signale mittels der Auswerteeinheit erfolgt analog zu den oben aufgeführten Ausführungsformen der erfindungsgemässen Vorrichtung.

Eine weitere vorteilhafte Ausführungsform der erfindungsgemässen Vorrichtung besteht darin, dass die zur Simulationseinrichtung gehörende Positioniereinrichtung zu einem Computer Assisted Surgery System (CAS) gehört, das mittels Vermessung von Objekten bezüglich Lage und Orientierung im Raum erlaubt, dass die Implantate in der erfindungsgemässen Vorrichtung so positioniert werden können, dass sie dieselbe Lage und Orientierung zueinander einnehmen wie die am Fixationssystem angebrachten. Ist dann die Position der Implantate und der Verlängerungsstifte in der erfindungsgemässen Vorrichtung identisch wie in situ wird ein Probestab respektive Biegeschablone in die dazu vorgesehenen Öffnungen an den Köpfen der Implantate in der erfindungsgemässen Vorrichtung gelegt und angepasst oder die Position der Implantate zueinander wird elektronisch vermessen und der Längsträger mechanisch angebogen.

Eine andere vorteilhafte Ausführungsform der erfindungsgemässen Vorrichtung besteht darin, dass die zur Simulationseinrichtung gehörende Positioniereinrichtung mechanisch nach dem Prinzip eines Pantographen arbeitet und ermöglicht, dass die Implantate in der erfindungsgemässen Vorrichtung so positioniert werden können, dass sie dieselbe Lage und Orientierung zueinander einnehmen wie in vivo am Fixationssystem angebracht. Ist dann die Position der Implantate und der Verlängerungsstifte in der erfindungsgemässen Vorrichtung identisch wie in situ wird ein Probestab respektive eine Biegeschablone in die dazu vorgesehenen Öffnungen an den Implantaten in der erfindungsgemässen Vorrichtung gelegt und angepasst oder die Position der Implantate zueinander wird elektronisch vermessen und der Längsträger mechanisch angebogen.

Wiederum eine andere vorteilhafte Ausführungsform der erfindungsgemässen Vorrichtung besteht darin, dass die zur erfindungsgemässen Vorrichtung gehörende Positioniereinrichtung ein mechanisches Abtastgerät umfasst. Dieses mechanische Abtastgerät ermöglicht, dass die Verlängerungsstifte und die Implantate in der erfindungsgemässen Vorrichtung so positioniert werden können, dass sie dieselbe Lage und Orientierung zueinander einnehmen wie die am Fixationssystem angebrachten. Ist dann die Position der Implantate und der Verlängerungsstifte in der erfindungsgemässen Vorrichtung identisch wie in situ wird ein Probestab respektive eine Biegeschablone in die dazu vorgesehenen Öffnungen an den Implantaten in der erfindungsgemässen Vorrichtung gelegt und angepasst oder die Position der Implantate zueinander wird elektronisch vermessen und der Längsträger mechanisch angebogen.

Wiederum eine andere vorteilhafte Ausführungsform der erfindungsgemässen Vorrichtung besteht darin, dass eine mechanisch anpassbare Übertragungsvorrichtung ermöglicht, dass über die Positionierstifte und Hülsen durch Anpassen an die Implantate und Verlängerungsstifte des Fixationssystems diese so eingestellt werden können, dass durch Verlagerung der Übertragungsvorrichtung auf die in der Vorrichtung eingespannten Verlängerungsstifte und Implantate diese in dieselbe Lage und Orientierung zueinander gebracht werden können wie die zum Fixationssystem gehörenden Implantate und Verlängerungsstifte. Ist dann die Position der Implantate und der Verlängerungsstifte in der erfindungsgemässen Vorrichtung identisch wie in situ wird ein Probestab respektive eine Biegeschablone in die dazu vorgesehenen Öffnungen an den Implantaten in der erfindungsgemässen Vorrichtung gelegt und angepasst oder die Position der Implantate zueinander wird elektronisch vermessen und der Längsträger mechanisch angebogen.

Eine weitere vorteilhafte Ausführungsart der erfindungsgemässen Vorrichtung besteht darin, dass sie zusätzlich ein Klemmvorrichtung umfasst, welche eine zur Länge des Längsträgers anpassbare Längenanzeige enthält und ermöglicht, dass der vorgebogene Längsträger unter die Haut bzw. unter die Muskulatur geschoben und in die dafür vorgesehenen Öffnungen an den Implantaten gebracht wird. Die Längenanzeige an der Klemmvorrichtung verläuft über der Haut parallel zum vorgebogenen Längsträger und dient somit als Orientierungshilfe.

In einer vorteilhaften Ausführungsart des erfindungsgemässen Verfahrens zum Anbiegen eines Längsträgers, welcher mehrere Implantate innerhalb eines Fixationssystems verbindet, werden an die Implantate in situ Verlängerungsstifte angebracht. Die die Form des Längsträgers bestimmende Lage und Orientierung dieser Implantate und Verlängerungsstifte zueinander wird mittels einer Positioniereinrichtung auf identische Implantate und Verlängerungsstifte übertragen, wodurch die die Form des Längsträgers bestimmende Lage und Orientierung der Implantate simuliert wird. Der Längsträger wird dort an die Implantate angepasst und anschliessend implantiert.

Eine weitere vorteilhafte Ausführungsart des erfindungsgemässen Verfahrens besteht darin, dass die die Form des Längsträgers bestimmende Lage und Orientierung der am Fixationssystem angebrachten Implantate und Verlängerungstifte mittels einer Positioniereinrichtung auf identische, in einer Vorrichtung eingespannte Implantate und Verlängerungsstifte übertragen wird. Diese Vorrichtung gestattet auch, zu simulieren, wie die Anatomie verändert wird, wenn der Längsträger mit leichten Veränderungen gegenüber der durch die Implantate in situ vorgegebenen Form gebogen wird. Ebenfalls simulieren lassen sich Veränderungen der Anatomie, welche durch Veränderung der Position der Implantate erreicht werden.

Die durch die Erfindung erreichten Vorteile sind im wesentlichen darin zu sehen, dass:
A) die Weichteile nicht geöffnet werden müssen, um die Kontur des erforderlichen Längsträgers zu bestimmen;
B) der Probestab respektive Biegeschablone nicht mit Behinderung durch die Weichteile in der Tiefe in das Implantat eingebracht und wieder herausgenommen wird, so dass Gefahr besteht, dass sich der Probestab respektive die Biegeschablone wieder verbiegt. Der Probestab respektive die Biegeschablone kann einfach in die erfindungsgemässe Vorrichtung eingebracht und bei Bedarf auch ohne Verbiegen wieder herausgenommen werden.
C) Ebenfalls erleichtert wird durch die erfindungsgemässe Vorrichtung die Kontrolle, ob der Längsträger richtig angebogen ist.
D) Auch eine eventuelle Korrektur durch das Anbiegen des Längsträgers selbst kann simuliert werden.

Die Erfindung und Weiterbildungen der Erfindung werden im folgenden anhand der teilweise schematischen Darstellungen mehrerer Ausführungsbeispiele noch näher erläutert.
Es zeigen:
Fig. 1 eine Ansicht von oben auf ein Stück der Wirbelsäule mit einem implantierten Längsträger sowie eine Ansicht von oben auf eine Ausführungsform der erfindungsgemässen Vorrichtung;
Fig. 2 einen Querschnitt durch eine Ausführungsform der erfindungsgemässen Vorrichtung;
Fig. 3 eine perspektivische Darstellung einer Ausführungsform der erfindungsgemässen Vorrichtung;
Fig. 4 eine perspektivische Darstellung einer weiteren Ausführungsform der erfindungsgemässen Vorrichtung;
Fig. 5 eine Klemmvorrichtung mit einer Längen- und Orientierungsanzeige zum Einbringen des Längsträgers unter die Haut bzw. unter die Muskulatur; und
Fig. 6 eine Ansicht der erfindungsgemässen Übertragungsvorrichtung.

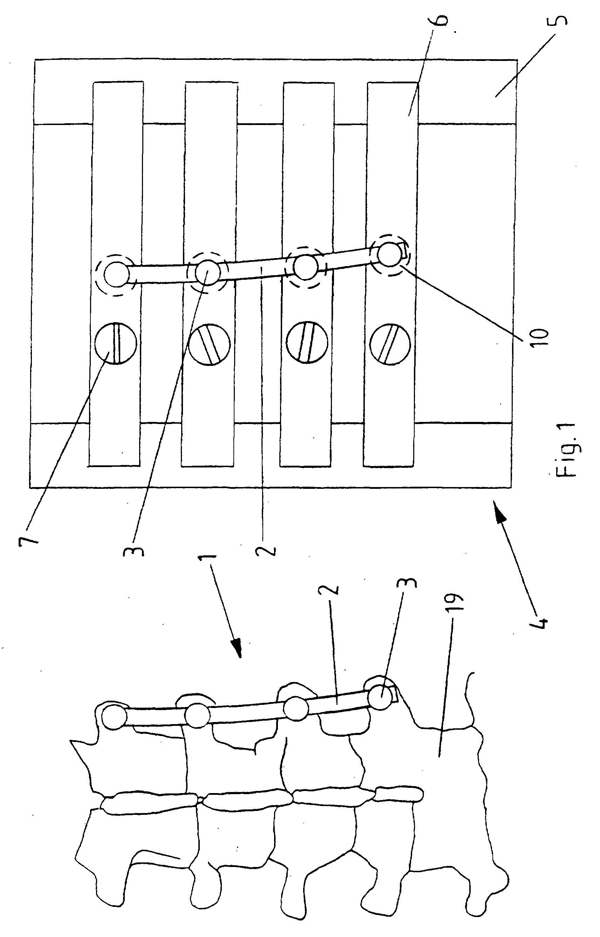

In Fig. 1 ist ein Stück einer Wirbelsäule mit einem Wirbelsäulenimplantat 1, welches mehrere Implantate 3 und einen Längsträger 2 enthält, und ebenfalls eine Variante der Vorrichtung 4 gezeigt. Die Vorrichtung 4 ist so eingestellt, dass der dort eingespannte Längsträger 2 die gleiche Gestalt annimmt wie derjenige im daneben gezeichneten Wirbelsäulenimplantat 1. Die gezeichnete Variante der Vorrichtung 4 zeigt wie die Implantate 3 in der von oben sichtbaren Ebene je am Wirbelsäulenimplantat 1 und in der Vorrichtung 4 die gleiche Stellung zueinander einnehmen. Die Verschiebungen der Implantate 3 in der Vorrichtung 4 wird durch ein Verschieben der Querträger 6 der erfindungsgemässen Vorrichtung erreicht. Die Querträger 6 lassen sich in den zwei Dimensionen der Ebene der Grundplatte 27 bewegen und durch Klemmschrauben 7 in einer beliebigen Position fixieren. In den Querträgern 6 sind Kugelgelenke 10 integriert. In diese Kugelgelenke 10 können gleiche Implantate 3 wie im Fixationssystem eingeschraubt werden. Diese Schraubbarkeit ermöglicht eine Bewegung des Implantates 3 in der dritten, senkrecht zur Rahmenebene stehenden Dimension während die Kugelgelenke 10 eine freie Drehung und damit Winkeleinstellung der Implantate 3 zulassen.

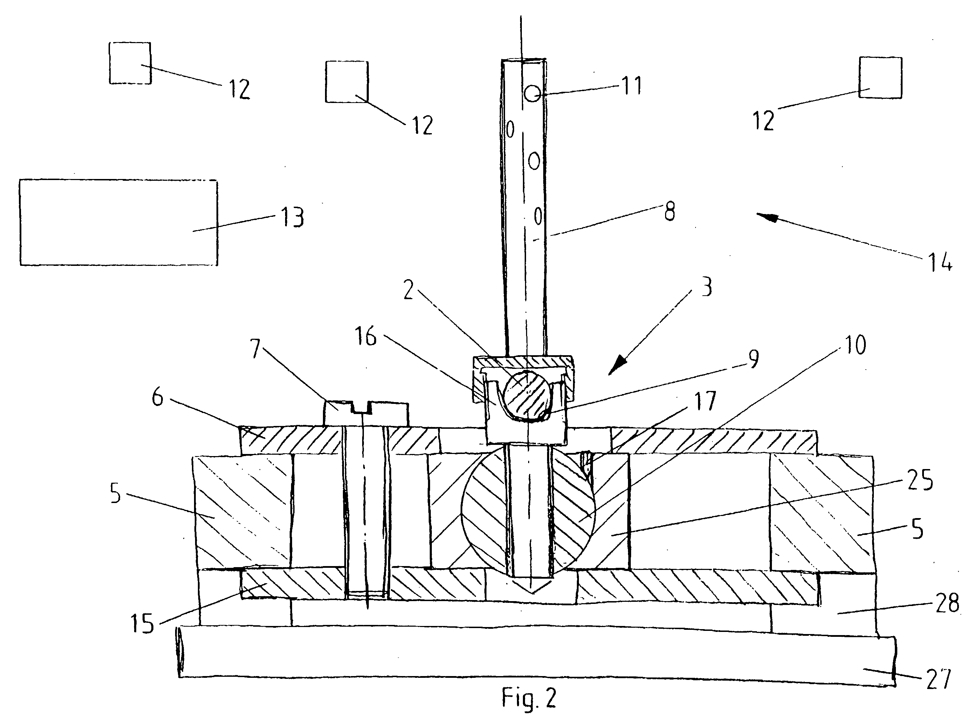

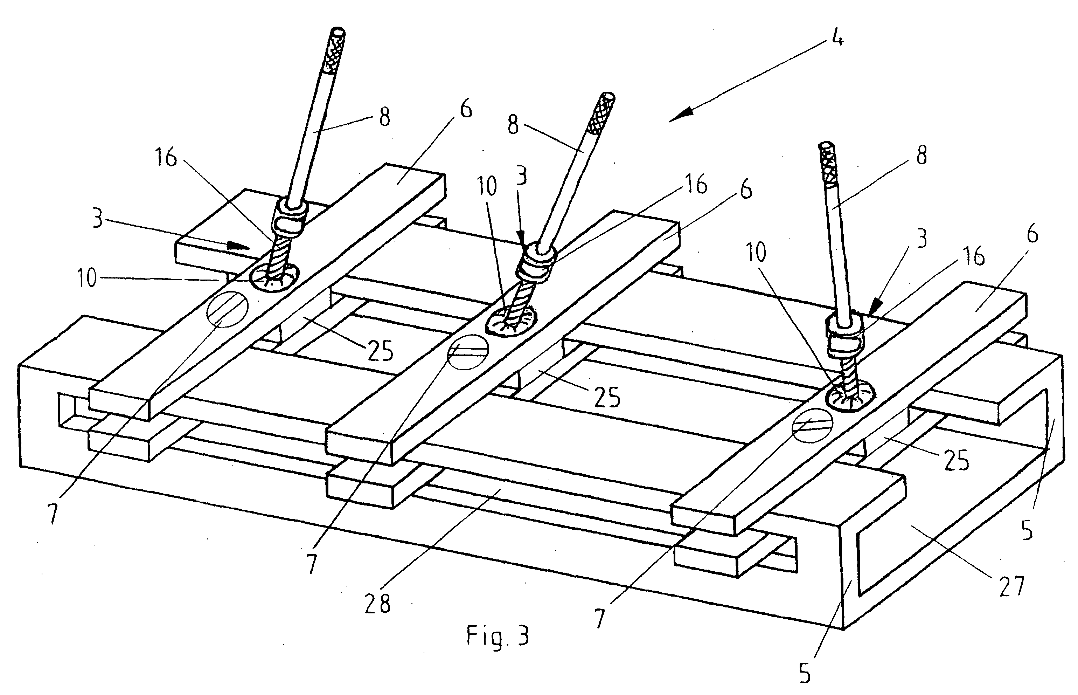

In Fig. 2 und 3 ist eine andere vorteilhafte Ausführungsform der erfindungsgemässen Vorrichtung gezeigt. Die Vorrichtung 4 umfasst eine Grundplatte 27 mit parallel angebrachten Längsschienen 5, Querträger 6 und eine Positioniereinrichtung 14. An die Querträger 6 angebracht sind ein Mittelstück 25 mit einem integrierten Kugelgelenk 10 und eine untere Platte 15. Die Querträger 6 lassen sich in den zwei Dimensionen der Grundplattenebene bewegen und durch Klemmschrauben 7 in einer beliebigen Position fixieren. In die Vorrichtung 4 werden dann die gleichen Implantate 3 mit gleichen Verlängerungsstiften 8 wie im Fixationssystem 1 in situ eingeschraubt. Eine zur Vorrichtung 4 gehörende Positioniereinrichtung 14 umfasst an den Verlängerungsstiften 8 angebrachte Light Emitting Dioden (LED) 11, mehrere diese emittierten elektromagnetischen Wellen detektierende Sensoren 12 und eine Auswerteeinheit 13. Die Auswerteeinheit 13 ermöglicht durch Verarbeitung von Interferenzbildern, dass die Verlängerungsstifte 8 und die Implantate 3 in der Vorrichtung 4 so positioniert werden können, dass sie dieselbe Lage und Orientierung zueinander einnehmen wie die am Fixationssystem 1 angebrachten Implantate und Verlängerungsstifte. Ist dann die Position der Implantate 3 und der Verlängerungsstifte 8 in der erfindungsgemässen Vorrichtung 4 identisch wie im Fixationssystem 1 wird ein Probestab respektive Biegeschablone in die dazu vorgesehenen Öffnungen 9 an den Köpfen 16 der Implantate 3 in der Vorrichtung 4 gelegt und angepasst oder die Position der Implantate 3 zueinander wird elektronisch vermessen und der Längsträger 2 mechanisch angebogen.

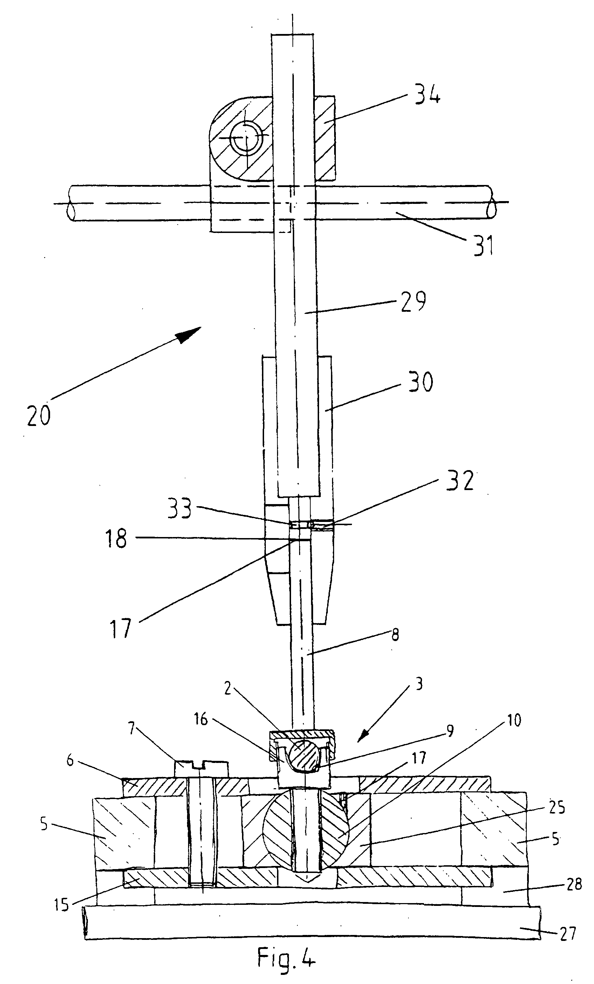

Fig. 4 zeigt eine weitere vorteilhafte Ausführungsform der erfindungsgemässen Vorrichtung. Die Vorrichtung 4 umfasst eine Grundplatte 27 mit parallel angebrachten Längsschienen 5, Querträger 6 und eine Positioniereinrichtung 14. An die Querträger 6 angebracht sind ein Mittelstück 25 mit einem integrierten Kugelgelenk 10 und eine untere Platte 15. Die Querträger 6 lassen sich in den zwei Dimensionen der Grundplattenebene bewegen und durch Klemmschrauben 7 in einer beliebigen Position fixieren. In die Vorrichtung 4 werden dann die gleichen Implantate 3 mit gleichen Verlängerungsstiften 8 wie im Fixationssystem 1 in situ eingeschraubt. Eine mechanisch anpassbare Übertragungsvorrichtung 20, welche Positionierstifte 29, Hülsen 30, Klemmelemente 34 und einen Verbindungsstab 31 umfasst, ermöglicht, dass die Positionierstifte 29 und Hülsen 30 durch Anpassen an die Implantate 3 und Verlängerungsstifte 8 des Fixationssystems 1 so eingestellt werden können, dass durch Verlagerung der Übertragungsvorrichtung 20 auf die in der Vorrichtung 4 eingespannten Verlängerungsstifte 8 und Implantate 3 diese in dieselbe Lage und Orientierung zueinander gebracht werden können wie die zum Fixationssystem 1 gehörenden Implantate 3 und Verlängerungsstifte 8. Die Positionierstifte 29 können mittels der Klemmelemente 34 räumlich frei drehbar und in einer gewählten Position fixierbar am Verbindungsstab 31 befestigt werden. Die Hülsen 30 ermöglichen dadurch, dass sie in axialer Richtung teilweise über die Verlängerungsstifte 8 an den Implantaten 3 geschoben werden können, dass die Positionierstifte 29 die axiale Verlängerung der Verlängerungsstifte 8 bilden, wobei die hinteren Stirnflächen 17 der Verlängerungsstifte 8 und die vorderen Stirnflächen 18 der Positionierstifte 29 genau aufeinander zu liegen kommen.

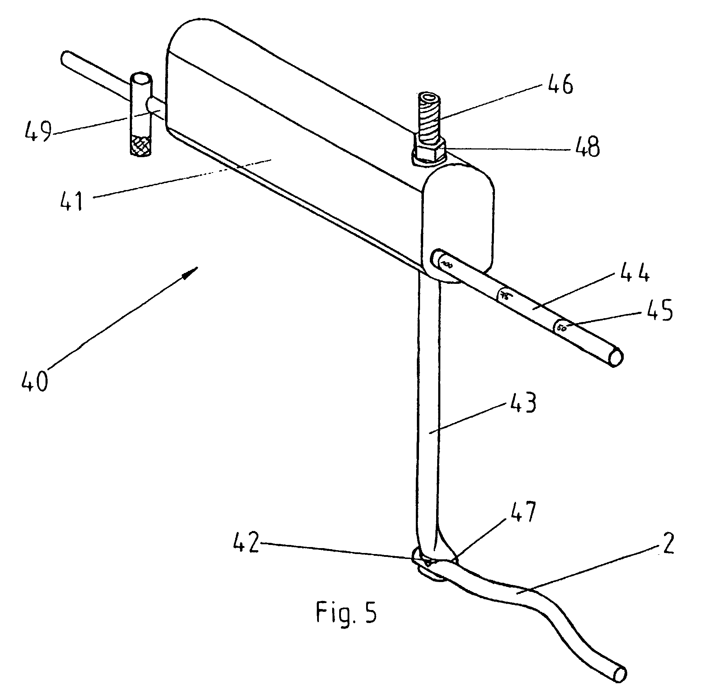

Fig. 5 zeigt eine Klemmvorrichtung 40 mit deren Hilfe der Längsträger 2 in die unter der Haut bzw. Muskulatur liegenden Implantate eingebracht werden kann. Die Klemmvorrichtung 40 umfasst einen Handgriff 41, eine Halterung 43 mit einer Aussparung 47 für den Längsträger 2, einen Klemmstift 42 und eine Längen- und Orientierungsanzeige 44 mit einer Längenskala 45. Die stabförmige Halterung 43 ist fest im Handgriff 41 angebracht und enthält an einem Ende eine Aussparung 47, die so beschaffen ist, dass der Längsträger 2, wenn er eingelegt wird, bezüglich seiner Längsachse eine bestimmte Orientierung einnimmt. Die Länge der Halterung 43 ist so bemessen, dass der Längsträger 2 unter die Haut bzw. Muskulatur geschoben werden kann während der Handgriff 41 ausserhalb des Körpers bleibt. Der in die Aussparung 47 eingelegte Längsträger 2 wird mittels eines Klemmstiftes 42, der axial in der Halterung 43 verschiebbar und durch ein Gewinde feststellbar ist, festgeklemmt. Parallel zum in der Aussparung 47 eingespannten Längsträger 2 ist im Handgriff 41 eine stabförmige Längen- und Orientierungsanzeige 44, welche parallel zum Längsträger 2 verschiebbar ist, gelagert. Mit Hilfe dieser Längen- und Orientierungsanzeige 44 lässt sich die Lage und Orientierung des einzubringenden Längsträgers 2 erkennen, wenn der Längsträger 2 zur Anpassung oder Implantation unter der Haut bzw. Muskulatur liegt. Der Längsträger 2 kann durch Lösen der Mutter 48 parallel zur Längen- und Orientierungsanzeige 44 ausgerichtet werden. Durch Lösen der Schraube 49 lässt sich die Länge der Längen- und Orientierungsanzeige 44 ausrichten.

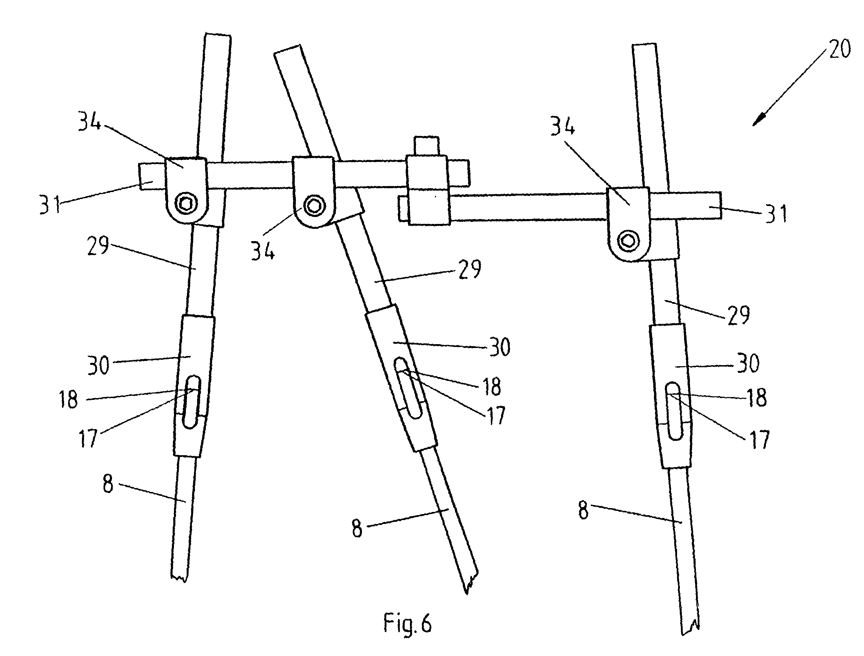

Fig. 6 zeigt eine Ausführungsform der mechanisch anpassbaren Übertragungsvorrichtung 20 mit deren Hilfe die gegenseitige Lage und Orientierung der Implantate 3 und Verlängerungsstifte 8 vom in Fig. 1 dargestellten Fixationssystem 1 auf die ebenfalls in Fig. 1 gezeigte Vorrichtung 4 übertragen werden kann. Die Übertragungsvorrichtung 20 umfasst Positionierstifte 29, teilweise über die Positionierstifte 29 schiebbare Hülsen 30, Klemmelemente 34 und einen Verbindungsstab 31, der hier aus zwei Stäben zusammengesetzt ist. Durch die Klemmelemente 34, welche axial am Verbindungsstab 31 verschoben werden können und um die Längsachse des Verbindungsstabes 31 drehbar sind, lassen sich die Positionierstifte 29 in zwei Achsenrichtungen verschieben und um zwei senkrecht zueinander liegende Drehachsen rotieren. Die Positionierstifte 29 und Hülsen 30 werden am Fixationssystem 1 bezüglich ihrer Lage und Orientierung eingestellt. Die Hülsen 30 ermöglichen dadurch, dass sie in axialer Richtung teilweise über die Verlängerungsstifte 8 an den Implantaten 3 geschoben werden können, dass die Positionierstifte 29 die axiale Verlängerung der Verlängerungsstifte 8 bilden können, wobei die hinteren Stirnflächen 17 der Verlängerungsstifte 8 und die vorderen Stirnflächen 18 der Positionierstifte 29 genau aufeinander zu liegen kommen.

## Patentansprüche

1. Vorrichtung (4) zur Simulation der
A) Lage und Orientierung von mehreren zu einem Fixationssystem (1) gehörenden Implantaten (3); und damit
B) der Form eines Längsträgers (2), der innerhalb des Fixationssystemes (1) zur Verbindung dieser Implantate (3) dient, **dadurch gekennzeichnet, dass**
C) die Vorrichtung (4) eine Grundplatte (27) mit parallel angebrachten Längsschienen (5) und auf diesen Längsschienen (5) bewegbare Querträger (6) umfasst und in diesen Querträgern (6) Kugelgelenke (10) integriert sind;
D) die Querträger (6) sich in den zwei Dimensionen der Ebene der Grundplatte (27) bewegen und durch Klemmschrauben (7) in einer beliebigen Position fixieren lassen;
E) in diese Kugelgelenke (10) gleiche Implantate (3) mit gleichen Verlängerungsstiften (8) wie im Fixationssystem (1) einschraubbar sind, wodurch sich auch die Höhe der Implantate (3) zueinander einstellen lässt, und die Kugelgelenke (10) die freie Drehung und damit Winkeleinstellung der Implantate (3) gewährleisten;
F) eine Positioniereinrichtung (14) vorgesehen ist, mit welcher die Verlängerungsstifte (8) und die Implantate (3) in der Vorrichtung (4) so positionierbar sind, dass sie dieselbe Lage und Orientierung zueinander einnehmen wie die am Fixationssystem (1) angebrachten Implantate.

2. Vorrichtung nach Anspruch 1, **dadurch gekennzeichnet, dass** die Positioniereinrichtung (14) mit Hilfe von an den Implantaten (3) oder Verlängerungsstiften (8) angebrachten, elektromagnetische oder akustische Wellen abgebenden oder reflektierenden Mitteln und einem diese Wellen empfangenden Sensorsystem arbeitet.

3. Vorrichtung nach Anspruch 1, **dadurch gekennzeichnet, dass** die Positioniereinrichtung (14) mit Hilfe von akustische oder elektromagnetische Wellen abgebenden Mitteln im Raum und an den Implantaten (3) oder Verlängerungsstiften (8) angebrachten, diese Wellen empfangenden Sensoren arbeitet.

4. Vorrichtung nach Anspruch 1, **dadurch gekennzeichnet, dass** die Positioniereinrichtung (14) durch mechanische Mittel die Übertragung der Lage und Orientierung der Implantate (3) und Verlängerungsstifte (8) zwischen dem Fixationssystem (1) und der Vorrichtung (4) oder umgekehrt ermöglicht.

5. Vorrichtung nach Anspruch 1 oder 2, **dadurch gekennzeichnet, dass** die Positioniereinrichtung (14) an den Verlängerungsstiften (8) angebrachte, elektromagnetische Wellen emittierende Sender (11), mindestens zwei die Interferenzmuster dieser elektromagnetischen Wellen detektierende Sensoren (12) und eine Auswerteeinheit (13) umfasst und die Auswerteeinheit (13) ermöglicht, die durch die Sensoren (12) detektierten Signale so zu repräsentieren, dass die in der Vorrichtung (4) eingespannten Verlängerungsstifte (8) und Implantate (3) in dieselbe Lage und Orientierung zueinander gebracht werden können wie die zum Fixationssystem (1) gehörenden Implantate (3) und Verlängerungsstifte (8) oder umgekehrt.

6. Vorrichtung nach Anspruch 1 oder 2, **dadurch gekennzeichnet, dass** die Positioniereinrichtung (14) an den Verlängerungsstiften (8) angebrachte, elektromagnetische Wellen emittierende Sender (11) enthält, das Sensorsystem der Positioniereinrichtung (14) Kameras enthält, sich mit Hilfe von diesen Kameras die von den Sendern (11) emittierten elektromagnetischen Wellen aufnehmen lassen und die Auswerteeinheit (13) mittels eines videogrammetrischen Verfahrens es ermöglicht, die durch die Kameras aufgenommenen Bilder so zu repräsentieren, dass die in der Vorrichtung (4) eingespannten Verlängerungsstifte (8) und Implantate (3) in dieselbe Lage und Orientierung zueinander gebracht werden können wie die zum Fixationssystem gehörenden Implantate (3) und verlängerungsstifte (8) oder umgekehrt.

7. Vorrichtung nach einem der Ansprüche 1 bis 3 oder 5 bis 6, **dadurch gekennzeichnet, dass** die Sender (11) optische Leuchtquellen sind.

8. Vorrichtung nach einem der Ansprüche 1 bis 3 oder 5 bis 6, **dadurch gekennzeichnet, dass** die Sender (11) Light Emitting Dioden (LED) sind.

9. Vorrichtung nach Anspruch 6, **dadurch gekennzeichnet, dass** die Kameras digitale Kameras sind.

10. Vorrichtung nach Anspruch 1 oder 2, **dadurch gekennzeichnet, dass** die Positioniereinrichtung (14) an den Verlängerungsstiften (8) angebrachte, Schallwellen emittierende Sender (11), mindestens zwei die Interferenzmuster dieser Schallwellen detektierende Sensoren (12) und eine Auswerteeinheit (13) umfasst und die Auswerteeinheit (13) ermöglicht, die durch die Sensoren (12) detektierten Signale so zu repräsentieren, dass die in der Vorrichtung (4) eingespannten Verlängerungsstifte (8) und Implantate (3) in dieselbe Lage und Orientierung zueinander gebracht werden können wie die zum Fixationssystem gehörenden Implantate (3) und Verlängerungsstifte (8) oder umgekehrt.

11. Vorrichtung nach Anspruch 1 oder 3, **dadurch gekennzeichnet, dass** die Positioniereinrichtung (14) mindestens zwei Schallwellen emittierende Sender im Raum und an den Verlängerungsstiften (8) angebrachte, mindestens zwei die Interferenzmuster dieser Schallwellen detektierende Sensoren und eine Auswerteeinheit (13) umfasst und die Auswerteeinheit (13) ermöglicht, die durch die Sensoren detektierten Signale so zu repräsentieren, dass die in der Vorrichtung (4) eingespannten Verlängerungsstifte (8) und Implantate (3) in dieselbe Lage und Orientierung zueinander gebracht werden können wie die zum Fixationssystem gehörenden Implantate (3) und Verlängerungsstifte (8) oder umgekehrt.

12. Vorrichtung nach einem der Ansprüche 1 bis 11, **dadurch gekennzeichnet, dass** die Positioniereinrichtung (14) Bestandteil einer zu einem bildverarbeitenden Computer Assisted Surgery System (CAS) gehörenden Vermessungseinrichtung von Lage und Orientierung ist.

13. Vorrichtung nach Anspruch 1 oder 4, **dadurch gekennzeichnet, dass** mit Hilfe einer mechanisch anpassbaren Übertragungsvorrichtung (20), welche Positionierstifte (29), Hülsen (30), Klemmelemente (34) und einen Verbindungsstab (31) umfasst, und die Positionierstifte (29) mittels der Klemmelemente (34) räumlich frei drehbar und in einer gewählten Position fixierbar am Verbindungsstab (31) befestigt werden können und die Hülsen (30) dadurch, dass sie in axialer Richtung teilweise über die Verlängerungsstifte (8) an den Implantaten (3) geschoben werden können, ermöglichen, dass die Positionierstifte (29) die axiale Verlängerung der Verlängerungsstifte (8) bilden, wobei die hinteren Stirnflächen (17) der Verlängerungsstifte (8) und die vorderen Stirnflächen (18) der Positionierstifte (29) genau aufeinander zu liegen kommen, die in der Vorrichtung (4) eingespannten Verlängerungsstifte (8) und Implantate (3) in dieselbe Lage und Orientierung zueinander gebracht werden können wie die zum Fixationssystem gehörenden Implantate (3) und Verlängerungsstifte (8) oder umgekehrt.

14. Vorrichtung nach Anspruch 1 oder 4, **dadurch gekennzeichnet, dass** die mechanischen Mittel einen Pantographen oder ein Abtastgerät oder einen Transporteur oder Kombinationen dieser Geräte umfassen.

15. Vorrichtung nach einem der Ansprüche 1 bis 14, **dadurch gekennzeichnet, dass** sie zusätzlich eine Klemmvorrichtung (40) umfasst, welche eine zur Länge des Längsträgers (2) anpassbare Längen- und Orientierungsanzeige (44) enthält und ermöglicht, dass der vorgebogene Längsträger (2) unter die Haut bzw. unter die Muskulatur geschoben und in die dafür vorgesehenen Öffnungen der Implantate (3) gebracht wird, während die Längen- und Orientierungsanzeige (44) über der Haut und somit sichtbar verbleibt.

16. Verfahren zum Anbiegen eines Längsträgers (2), in einer Vorrichtung (4), wobei der Längsträger geeignet ist, mehrere Implantate (3) in einem Fixationssystem (1) zu verbinden, **dadurch gekennzeichnet, dass** es die folgenden Schritte umfasst
A) Einschrauben von gleichen Implantaten (3) mit gleichen Vertängerungsstiften (8) wie im Fixationssystem (1) in Kugelgelenke (10), welche in einem auf parallelen Längsschienen (5) der Vorrichtung (4) verschiebbaren Querträger (6) integriert sind;
B) Einstellen der Höhe der Implantate (3) relativ zueinander durch entsprechend tiefes Einschrauben der Implantate (3) in die Kugelgelenke (10);
C) Positionieren der unter B) in die Kugelgelenke (10) eingeschraubten Implantate (3) mit den Verlängerungsstiften (8) durch Bewegen der Querträger (6) in den zwei Ebenen einer zur Vorrichtung (4) zählenden Grundplatte (27) mit dem Längsschienen (5) und durch Drehen der Implantate (3) mittels der Kugelgelenke (10) unter Anwendung einer Positioniervorrichtung (14) bis die Verlängerungsstifte (8) und die Implantate (3) dieselbe Lage und Orientierung einnehmen wie die am Fixationssystem (1) angebrachten, so dass dadurch die die Form des Längsträgers (2) bestimmende Lage und Orientierung der Implantate (3) simuliert wird; und
D) Anpassen eines Längsträgers (2) an die in der Vorrichtung (4) fixierten Implantate (3).

17. Verfahren nach Anspruch 16, **dadurch gekennzeichnet, dass** die Position der Implantate (3 ) zueinander elektronisch vermessen und der Längsträger (2) mechanisch angebogen wird.

18. Verfahren nach Anspruch 16, **dadurch gekennzeichnet, dass** durch die Vorrichtung (4) simuliert wird, wie die Anatomie verändert wird oder verändert werden kann, wenn der Längsträger (2) mit Veränderungen gegenüber der durch die Implantate (3) in situ vorgegebenen Form gebogen wird.

19. Verfahren nach Anspruch 16, **dadurch gekennzeichnet, dass** durch die Vorrichtung (4) simuliert wird, wie die Anatomie verändert wird oder verändert werden kann, wenn die Position eines Implantates (3) verändert wird.

## Claims

1. Device (4) for simulating
A) the position and orientation of several implants (3) belonging to one fixation system (1); and thus
B) the shape of a longitudinal rod (2) which serves to connect these implants (3) within the fixation system (1),
**characterized in that**
C) the device (4) comprises a base plate (27) with longitudinal splints (5) mounted in parallel and cross rods (6) that can be moved on these longitudinal splints (5) as well as ball joints (10) integrated into these cross rods (6);
D) the cross rods (6) can be moved in both dimensions of the plane of the base plate (27) and can be secured in any desired position by means of locking screws (7);
E) the same implants (3) can be screwed into these ball joints (10) with the same extension pins (8) as in the fixation system (1), so the height of the implants (3) relative to one another can be adjusted, and the ball joints (10) guarantee free rotation and thus the angular adjustment of the implants (3);
F) a positioning device (14) is provided with which the extension pins (8) and the implants (3) can be positioned in the device (4) so that they assume the same position and orientation relative to one another as those implants mounted on the fixation system (1).

2. Device according to Claim 1, **characterized in that** the positioning device (14) operates with the help of reflective means or means that emit electromagnetic or acoustic waves and are mounted on the implants (3) or on the extension pins (8) and with a sensor system to receive these waves.

3. Device according to Claim 1, **characterized in that** the positioning device (14) operates with the help of means that deliver acoustic or electromagnetic waves in space and sensors mounted on the implants (3) or extension pins (8) to receive these waves.

4. Device according to Claim 1, **characterized in that** the positioning device (14) permits a transfer of the position and orientation of the implants (3) and the extension pins (8) between the fixation system (1) and the device (4) or vice versa by mechanical means.

5. Device according to Claim 1 or 2, **characterized in that** the positioning device (14) comprises transmitters (11) that emit electromagnetic waves and are mounted on the extension pins (8), at least two sensors (12) which detect the interference patterns of these electromagnetic waves and an analyzing unit (13), and the analyzing unit (13) makes it possible to represent the signals detected by the sensors (12) that the extension pins (8) and implants (3) secured in the device (4) can be brought into the same position and orientation relative to one another as the implants (3) and extension pins (8) belonging to the fixation system (1).

6. Device according to Claim 1 or 2, **characterized in that** the positioning device (14) comprises transmitters (11) which are mounted on the extension pins (8) and emit electromagnetic waves; the sensor system of the positioning device (14) has cameras; the electromagnetic waves emitted by the transmitters (11) can be recorded with the help of these cameras, and the analyzing unit (13) makes it possible by means of a video analysis method to represent the images recorded by the cameras in such a way that the implants (3) and extension pins (8) secured in the device (4) can be brought into the same position and orientation relative to one another as the implants (3) and extension pins (8) belonging to the fixation system (1) or vice versa.

7. Device according to one of Claims 1 through 3 or 5 through 6, **characterized in that** the transmitter s (11) are optical light sources.

8. Device according to one of Claims 1 through 3 or 5 through 6, **characterized in that** the transmitters (11) are light emitting diodes (LEDs).

9. Device according to Claim 6, **characterized in that** the cameras are digital cameras.

10. Device according to Claim 1 or 2, **characterized in that** the positioning device (14) comprises transmitters (11) which are mounted on the extension pins (8) and emit sound waves, at least two sensors (12) which detect the interference patterns of these sound waves and an analyzing unit (13), and the analyzing unit (13) makes it possible to represent the signals detected by the sensors (12) in such a way that the implants (3) and extension pins (8) secured in the device (4) can be brought into the same position and orientation relative to one another as the implants (3) and extension pins (8) belonging to the fixation system or vice versa.

11. Device according to Claim 1 or 3, **characterized in that** the positioning device (14) comprises at least two transmitters that emit sound waves in space, at least two sensors that detect the interference patterns of these sound waves and are mounted on the extension pins (8), and an analyzing unit (13), representing the signals detected by the sensors so that the implants (3) and extension pins (8) secured in the device (4) can be brought into the same position and orientation relative to one another as the implants (3) and extension pins (8) belonging to the fixation system or vice versa.

12. Device according to one of Claims 1 through 11, **characterized in that** the positioning device (14) is part of a device for measuring the position and orientation and belongs to an image processing computer assisted surgery system (CAS).

13. Device according to Claim 1 or 4, **characterized in that** with the help of a mechanically adjustable transfer device (20) which comprises positioning pins (29), sleeves (30), clamping elements (34) and a connecting rod (31), and the positioning pins (29) which can rotate freely in space by means of the clamping elements (34) and can be secured in a selected position on the connecting rod (31), and due to the fact that the sleeves (30) can be pushed in the axial direction partially over the extension pins (8) on the implants (3), they make it possible for the positioning pins (29) to form the axial extension of the extension pins (8), where the rear end faces (17) of the extension pins (8) and the front end faces (18) of the positioning pins (29) come to lie precisely in contact with one another, so that the implants (3) and extension pins (8) secured in the device (4) can be brought into the same position and orientation relative to one another as the implants (3) and extension pins (8) belonging to the fixation system or vice versa.

14. Device according to Claim 1 or 4, **characterized in that** the mechanical means comprise a pantograph or a scanning device or a transporter or combinations of these devices.

15. Device according to one of Claims 1 through 14, **characterized in that** it also includes a clamping device (40) which has a position and orientation display (44) that can be adjusted to the length of the longitudinal rod (2) and makes it possible for the prebent longitudinal rod (2) to be inserted beneath the skin or the muscles and brought into the openings provided for this purpose in the implants (3) while the position and orientation display (44) remains above the skin and is thus visible.

16. Method of prebending a longitudinal rod (2) in a device (4), whereby the longitudinal rod (2) is apt to connect several implants (3) within a fixation system (1), **characterized in that** it comprises the following steps:
A) mounting of idendical implants (3) with identical extension pins (8) as in the fixations system (2) in ball joints (10), that are integrated in cross rods (6) that can be moved on parallel longitudinal splints (5) of the device (4);
B) adjusting of the height of the implants (3) relative to each other through screwing the implants (3) in the ball joints (10) to an appropriate depth;
C) positioning of the implants (3) together with the extension pins (8) being screwed into the ball joints (10) under step B) by means of moving the cross rods (6) in the plane of a base plate (27) with the longitudinal splints (5) being part of the device (4) and by means of pivoting the implants (3) by means of the ball joints (10) using a positioning device (14) unless the position and orientation of these implants (3) corresponds to those being mounted on the fixation system (1), thus simulating the position and orientation of the implants (3) which determines the shape of the longitudinal rod (2); and
D) adapting the longitudinal rod (2) to the implants (3) being fixed in the device (4).

17. Method according to Claim 16, **characterized in that** t the position of the implants (3) relative to one another is determined electronically and the longitudinal rod (2) is prebent mechanically.

18. Method according to Claim 16, **characterized in that** the device (4) simulates how the anatomy changes or can be changed when the longitudinal rod (2) is bent with changes in comparison with the shape predetermined by the implants (3) in situ.

19. Method according to Claim 16, **characterized in that** the device (4) simulates how the anatomy changes or can be changed when the position of an implant (3) is changed.

## Revendications

1. Dispositif (4) de simulation de
A) la position et l'orientation de plusieurs implants (3) faisant partie d'un système de fixation (1), et ainsi
B) la forme d'un support longitudinal (2) qui sert à relier ces implants (3) au sein du système de fixation (1),
**caractérisé en ce que**
C) le dispositif (4) comprend une plaque de base (27) avec des rails longitudinaux (5) placés parallèlement et des traverses (6) mobiles sur ces rails longitudinaux (5), et des articulations sphériques (10) sont intégrées dans ces traverses (6),
D) les traverses (6) se meuvent dans les deux dimensions du plan de la plaque de base (27) et peuvent être fixées dans une position quelconque par des vis de serrage (7),
E) des implants identiques (3) avec des tiges de prolongement (8) identiques peuvent être vissés dans ces articulations sphériques (10) comme dans le système de fixation (1), ce qui permet de régler la hauteur des implants (3) les uns par rapport aux autres, et les articulations sphériques (10) garantissent la libre rotation et donc le réglage angulaire des implants (3),
F) il est prévu un dispositif de positionnement (14) avec lequel les tiges de prolongement (8) et les implants (3) peuvent être positionnés dans le dispositif (4) de telle manière qu'ils prennent la même position et orientation les uns par rapport aux autres que les implants placés sur le système de fixation (1).

2. Dispositif selon la revendication 1, **caractérisé en ce que** le dispositif de positionnement (14) fonctionne à l'aide de moyens émettant ou réfléchissant des ondes électromagnétiques ou acoustiques, placés sur les implants (3) ou tiges de prolongement (8) et un système de capteur recevant les ondes.

3. Dispositif selon la revendication 1, **caractérisé en ce que** le dispositif de positionnement (14) fonctionne à l'aide de moyens émettant des ondes électromagnétiques ou acoustiques dans l'espace et de capteurs recevant ces ondes, placés sur les implants (3) ou tiges de prolongement (8).

4. Dispositif selon la revendication 1, **caractérisé en ce que** le dispositif de positionnement (14) permet, par des moyens mécaniques, la transmission de la position et orientation des implants (3) et tiges de prolongement (8) entre le système de fixation (1) et le dispositif (4).

5. Dispositif selon la revendication 1 ou 2, **caractérisé en ce que** le dispositif de positionnement (14) comprend des émetteurs (11) émettant des ondes électromagnétiques, placés sur les tiges de prolongement 8, au moins deux capteurs (12) détectant les franges d'interférence de ces ondes électromagnétiques et une unité d'analyse (13), et l'unité d'analyse (13) permet de représenter les signaux détectés par les capteurs (12) de telle manière que les tiges de prolongement (8) et implants (3) montés dans le dispositif (4) puissent être amenés dans la même position et orientation les uns par rapport aux autres que les implants (3) et tiges de prolongement (8) appartenant au système de fixation (1) ou vise-versa.

6. Dispositif selon la revendication 1 ou 2, **caractérisé en ce que** le dispositif de positionnement (14) comprend des émetteurs (11) émettant des ondes électromagnétiques, placés sur les tiges de prolongement 8, le système de capteurs du dispositif de positionnement (14) comprend des caméras, les ondes électromagnétiques émises par les émetteurs (11) peuvent être enregistrées à l'aide de ces caméras et l'unité d'analyse (13) permet au moyen d'un procédé vidéogrammatique de représenter les images enregistrées par les caméras de telle manière que les tiges de prolongement (8) et implants (3) montés dans le dispositif (4) puissent être amenés dans la même position et orientation que les implants (3) et tiges de prolongement (8) appartenant au système de fixation ou vice-versa.

7. Dispositif selon une des revendications 1 à 3 ou 5 à 6, **caractérisé en ce que** les émetteurs (11) sont des sources lumineuses optiques.

8. Dispositif selon une des revendications 1 à 3 ou 5 à 6, **caractérisé en ce que** les émetteurs (11) sont des diodes électroluminescentes (LED).

9. Dispositif selon la revendication 6, **caractérisé en ce que** les caméras sont des caméras numériques.

10. Dispositif selon la revendication 1 ou 2, **caractérisé en ce que** le dispositif de positionnement (14) comprend des émetteurs (11) émettant des ondes sonores, placés sur les tiges de prolongement 8, au moins deux capteurs (12) détectant les franges d'interférence de ces ondes sonores et une unité d'analyse (13), et l'unité d'analyse (13) permet de représenter les signaux détectés par les capteurs (12) de telle manière que les tiges de prolongement (8) et implants (3) montés dans le dispositif (4) puissent être amenés dans la même position et orientation les uns par rapport aux autres que les implants (3) et tiges de prolongement (8) appartenant au système de fixation (1) ou vise-versa.

11. Dispositif selon la revendication 1 ou 3, **caractérisé en ce que** le dispositif de positionnement (14) comprend au moins deux émetteurs (11) émettant des ondes sonores dans l'espace et placés sur les tiges de prolongement (8), au moins deux capteurs détectant les franges d'interférence de ces ondes sonores et une unité d'analyse (13), et l'unité d'analyse (13) permet de représenter les signaux détectés par les capteurs de telle manière que les que les tiges de prolongement (8) et implants (3) montés dans le dispositif (4) puissent être amenés dans la même position et orientation les uns par rapport aux autres que les implants (3) et tiges de prolongement (8) appartenant au système de fixation (1) ou vise-versa.

12. Dispositif selon une des revendications 1 à 11, **caractérisé en ce que** le dispositif de positionnement (14) est une partie constitutive d'un dispositif de mesure de la position et de l'orientation faisant partie d'un système de chirurgie assistée par ordinateur (CAO) traitant les images.

13. Dispositif selon la revendication 1 ou 4, **caractérisé en ce qu'**à l'aide d'un dispositif de transmission (20) adaptable mécaniquement, qui comprend des tiges de positionnement (29), des douilles (30), des éléments de serrage (34) et une barre de connexion (31), et les tiges de positionnement (29) peuvent être fixées au moyen des éléments de serrage (34) de manière librement rotative dans l'espace et de manière blocable dans une position choisie sur la barre de connexion (31) et les douilles (30), du fait qu'elles peuvent être déplacées dans le sens axial en partie par-dessus les tiges de prolongement (8) sur les implants (3), permettent que les tiges de positionnement (29) forment le prolongement axial des tiges de prolongement (8), les faces frontales arrière (17) des tiges de prolongement (8) et les faces frontales avant (18) des tiges de positionnement (29) venant reposer avec précision les unes sur les autres, les tiges de prolongement (8) et implants (3) montés dans le dispositif (4) peuvent être amenés dans la même position et orientation les uns par rapport aux autres que les implants (3) et tiges de prolongement (8) faisant partie du système de fixation ou vice-versa.

14. Dispositif selon la revendication 1 ou 4, **caractérisé en ce que** les moyens mécaniques comprennent un pantographe ou un appareil de balayage ou un transporteur ou des combinaisons de ces appareils.

15. Dispositif selon une des revendications 1 à 14, **caractérisé en ce qu'**il comprend en outre un dispositif de serrage (40) qui contient un indicateur de longueur et d'orientation (44) adaptable à la longueur du support longitudinal (2) et permet que le support longitudinal (2) pré-plié soit poussé sous la peau ou sous les muscles et placé dans les ouvertures des implants (3) prévues à cet effet, tandis que l'indicateur de longueur et d'orientation (44) reste au-dessus de la peau et donc visible.

16. Procédé pour plier un support longitudinal (2) dans un dispositif (4), le support longitudinal (2) étant apte à relier plusieurs implants (3) dans un système de fixation (1), **caractérisé en ce qu'**il comprend les étapes suivantes :
A) visser les mêmes implants (3) avec les mêmes tiges de prolongement (8) que dans le système de fixation (1) dans les articulations sphériques (10) qui sont intégrées dans une traverse (6) pouvant être déplacée sur des rails longitudinaux (5) parallèles du dispositif (4),
B) régler la hauteur des implants (3) les uns par rapport aux autres par un vissage en profondeur correspondant des implants (3) dans les articulations sphériques (10),
C) positionner les implants (3) vissés dans les articulations sphériques (10) sous B) avec les tiges de prolongement (8) en mouvant les traverses dans les deux plans d'une plaque de base (27) faisant partie avec les rails longitudinaux (5) du dispositif (4) et en faisant tourner les implants (3) au moyen des articulations sphériques (10) en utilisant un dispositif de positionnement (14) jusqu'à ce que les tiges de prolongement (8) et les implants (3) prennent la même position et orientation que ceux placés sur le système de fixation (1) afin que de ce fait la position et l'orientation des implants déterminant la forme du support longitudinal (2) soient simulée, et
D) adapter un support longitudinal (3) aux implants (3) fixés dans le dispositif (4).

17. Procédé selon la revendication 16, **caractérisé en ce que** la position des implants (3) les uns par rapport aux autres est mesurée électroniquement et le support longitudinal (2) est plié mécaniquement.

18. Procédé selon la revendication 16, **caractérisé en ce que** le dispositif permet de simuler la manière dont l'anatomie est modifiée ou peut être modifiée quand le support longitudinal (2) est plié avec des modifications par rapport à la forme prédéterminée in situ par les implants (3).

19. Procédé selon la revendication 16, **caractérisé en ce que** le dispositif permet de simuler la manière dont l'anatomie est modifiée ou peut être modifiée quand la position des implants (3) est modifiée.
